# EUROPÄISCHE PATENTSCHRIFT

(11) **EP 3 446 101 B1**
(45) Veröffentlichungstag und Bekanntmachung des Hinweises auf die Patenterteilung: **27.05.2020**
(21) Anmeldenummer: 17721550.6
(22) Anmeldetag: 19.04.2017
(51) Int. Cl.: G01N 21/64, G01N 21/94, B65D 79/02, G01N 33/02

(54) **CHARAKTERISIERUNG EINER OBERFLÄCHE EINES ARBEITSMITTELS WÄHREND EINER PRODUKTVORBEREITUNG UND/ODER PRODUKTVERPACKUNG**
CHARACTERIZATION OF A SURFACE OF AN EQUIPMENT DURING A PRODUCT PREPARATION AND/OR PRODUCT PACKAGING
CARACTÉRISATION D'UNE SURFACE D'ÉQUIPEMENT LORS DE LA PRÉPARATION ET/OU DE L'EMBALLAGE D'UN PRODUIT

(30) Priorität: 19.04.2016 DE 102016206600; 25.04.2016 DE 102016206951
(43) Veröffentlichungstag der Anmeldung: 27.02.2019
(73) Patentinhaber: GEA Food Solutions Germany GmbH, 35216 Biedenkopf-Wallau (DE)
(72) Erfinder: BRUNNQUELL, Norbert, 87452 Altusried (DE); HÜBNER, Gerd Martin, 57234 Wilnsdorf (DE); SASSMANNSHAUSEN, Volker, 57334 Bad Laasphe (DE); WEIDLICH, Manfred, 58456 Witten (DE)
(74) Vertreter: Kutzenberger Wolff & Partner
(86) Internationale Anmeldenummer: PCT/EP2017/059283
(87) Internationale Veröffentlichungsnummer: WO 2017/182519

(56) Entgegenhaltungen:
- CN-A- 105 419 986
- DE-A1-102006 006 220
- DE-A1-102012 105 291
- DE-B4-102013 008 003
- US-A1- 2004 171 137
- US-A1- 2006 121 165

## Beschreibung

Die vorliegende Erfindung betrifft ein Verfahren zum Verarbeiten und/oder Verpacken eines Lebensmittelproduktes oder eines medizintechnischen oder pharmakologischen Produkts und/oder anderen Hygiene sensitiven Produkts mit einer Verarbeitungs-, Handhabungs- und/oder Verpackungsmaschine.

Noch ein Gegenstand der vorliegenden Erfindung ist eine Verarbeitungs-, insbesondere Lebensmittelverarbeitungs-und/oder Verpackungsmaschine sowie ein Messgerät zum Messen eines Oberflächenbelags an Bakterien und/oder deren Stoffwechselprodukten und/oder von biochemischen Reaktionsprodukten auf einem Lebensmittel und/oder auf einer Verarbeitungs-, insbesondere Lebensmittelverarbeitungs- und/oder auf einer Verpackungsmaschine.

Bei der Verarbeitung und Verpackung von Lebensmitteln werden immer höhere Standards an die Hygiene des Lebensmittels gestellt. Dafür sind aus dem Stand der Technik Patente bzw. Patentanmeldungen, beispielsweise die DE 102013008003 B4, die DE 102011075667 A1 sowie die DE 19638693 A1, bekannt, die sich mit der Messung von Belägen, insbesondere von Stoffwechselprodukten von Bakterien auf Lebensmitteln beschäftigen, um damit auf eine Bakterienpopulation und folglich auf die Hygiene des Produkts zu schließen. Aus DE102006006220 A1 ist eine Verpackungsmaschine bekannt, wobei ein Belag der Verpackungsmaschine desinfiziert ist.

Es ist die Aufgabe der vorliegenden Erfindung die Hygiene der Lebensmittelverarbeitung bzw. -verpackung weiter zu verbessern.

Gelöst wird die Aufgabe mit einem Verfahren zum Verarbeiten und/oder Verpacken eines Lebensmittelproduktes oder eines medizintechnischen oder pharmakologischen Produkts und/oder Hygiene sensitiven Produkts mit einer Verarbeitungs-, Handhabungs- und/oder Verpackungsmaschine, bei dem ein Belag der Oberfläche des Lebensmittelproduktes oder des medizintechnischen oder pharmakologischen Produkts und/oder Hygiene sensitiven Produkts und/oder der Verarbeitungs- und/oder Verpackungsmaschine und/oder der Schutzkleidung eines Werkers mit Bakterien und/oder deren Stoffwechselprodukten und/oder von biochemischen Reaktionsprodukten mit einer UV-Strahlung angeregt wird, die fluoreszierende Rückstrahlung des Belages gemessen wird und die erzielten Messergebnisse in einem Speichermedium, das sich an einer Verpackung befindet, und/oder in einer Datenbank zusammen mit einer Verpackungsidentifikation abgespeichert werden.

Die zu diesem Gegenstand der vorliegenden Erfindung gemachten Ausführungen gelten für die anderen Gegenstände der vorliegenden Erfindung gleichermaßen und umgekehrt.

Im Zusammenhang mit der Erfindung ist ein Verfahren zum Reinigen einer Verarbeitungs-, insbesondere Lebensmittelverarbeitungs- und/oder Verpackungs-, insbesondere Lebensmittelverpackungsmaschine, bei dem vor und/oder während und/oder nach dem Reinigen der Belag der Verarbeitungs- und/oder Verpackungsmaschine mit Bakterien und/oder deren Stoffwechselprodukten gemessen wird.

Im Zusammenhang mit der Erfindung ist ein Verfahren zum Reinigen einer Verarbeitungs-, insbesondere Lebensmittelverarbeitungs- und/oder Verpackungs-, insbesondere Lebensmittelverpackungsmaschine, bei dem beim Erreichen oder Überschreiten eines zuvor festgelegten Grenzwertes der Anzahl von Bakterien und/oder der Menge von deren Stoffwechselprodukten und/oder der Anwesenheit von zuvor festgelegten, beispielsweise besonders gefährlichen und/oder unerwünschten Bakterien die Verarbeitungs-, insbesondere Lebensmittelverarbeitungs- und/oder Verpackungs-, insbesondere Lebensmittelverpackungsmaschine angehalten und/oder an einer Visualisierungseinheit ein kritischer Hygienezustand anzeigt wird und/oder das Reinigen der Maschine empfiehlt. Vorzugsweise kann die Maschine erst wieder angefahren werden, wenn das Reinigen der Maschine besonders quittiert wird.

Im Zusammenhang mit der Erfindung ist ein Verfahren zum Reinigen der Schutzkleidung eines Werkers, dadurch gekennzeichnet, dass vor und/oder während und/oder nach dem Reinigen der Belag der Schutzkleidung mit Bakterien und/oder deren Stoffwechselprodukten gemessen wird. Vorzugsweise wird der Werker nach erfolgter Messung über den Hygienezustand seiner Schutzkleidung informiert, beispielsweise durch ein akustisches oder visuelles, z. B. Licht-Signal und/oder einen Hinweistext, in dem der Werker beispielsweise zum Wechseln der Schutzkleidung aufgefordert wird.

Im Zusammenhang mit der Erfindung ist ein Verfahren zum Reinigen der Schutzkleidung eines Werkers, dadurch gekennzeichnet, dass vorzugsweise zyklisch, beispielsweise stündlich, die Farbe oder ein anderes hygienerelevantes Erkennungsmerkmal der Schutzkleidung des Werkers festgestellt, beispielsweise mit einem Farberkennungssensor gemessen wird und der Werker darauf aufmerksam gemacht wird, beispielsweise durch einen Warnhinweis an der Bedieneinheit und/oder einen Vibrationsalarm an einem mobilen Warngerät, das der Werker mitführt, dass das hygienegerechte Wechseln der Schutzkleidung beispielsweise unmittelbar bevorsteht oder sofort erledigt werden muss.

Ein Lebensmittel im Sinne der Erfindung ist jedes pflanzliche oder tierische Lebensmittel. Insbesondere ist das Lebensmittel proteinhaltig. Beispielsweise handelt es sich bei dem Lebensmittel um Fleisch, Fisch oder Käse. Aber auch deren Verarbeitungsprodukte wie Wurst, oder paniertes und/oder wärmebehandeltes Fleisch oder wärmebehandelter Fisch sind ein Lebensmittel im Sinne der Erfindung.

Ein medizintechnischer oder pharmakologischer Gegenstand im Sinne der Erfindung ist beispielsweise ein Operationsbesteck, eine Spritze oder ein Flüssigkeitsbeutel, sowie eine Tablette, Pulver, Salbe oder Creme.

Eine andere Hygiene sensitives Produkt ist beispielsweise eine Farbflüssigkeit, die z. B. für Inkjet-Patronen für Drucker vorgesehen ist und die beispielsweise nicht mit Bakterien kontaminiert werden soll und/oder verderblich ist.

Ein Verfahren zum Bearbeiten von Lebensmitteln umfasst beispielsweise:
- das Auftauen/Einfrieren von einem Lebensmittel,
- das Marinieren von einem Lebensmittel, bei dem die Oberfläche und oder das Innere des Lebensmittels mit einer Marinade behandelt wird,
- die mechanische Behandlung eines Lebensmittels, beispielsweise das Tumbeln und/oder Mixen und/oder Rütteln von Lebensmitteln,
- das Panieren von Lebensmitteln,
- die Wärmebehandlung von Lebensmittels, beispielsweise in einem Ofen oder einer Fritteuse,
- das Zerkleinern von Lebensmitteln, beispielsweise in einem Kutter oder Fleischwolf und/oder das Schneiden eines Lebensmittels in Scheiben oder Würfel,
- das Transportieren von Lebensmitteln, beispielsweise mit einem Transportband und/oder einem Transportwagen und/oder einem Roboter, vorzugsweise einem sogenannten Tripod, mit dem Lebensmittel oder medizintechnische oder pharmazeutische/pharmakologische Gegenstände von einem Zuführ-Transportband abgenommen und in die Verpackungen in einer Verpackungsmaschine eingelegt werden.

Ein Verfahren zum Verpacken von Lebensmitteln umfasst insbesondere den hermetischen, luftdichten Abschluss des Lebensmittels in einem Verpackungsmaterial, insbesondere in einer Verpackungsfolie. Insbesondere erfolgt das Verpacken mit einer Form-Füll-Siegelverpackungsmaschine, beispielsweise einem Thermoformer, einem Traysealer oder einer Schlauchbeutelmaschine.

Die Schutzkleidung eines Werkers umfasst beispielsweise dessen Schuhe, Schürze aber insbesondere dessen Handschuhe.

Der Erfindung liegt die Erkenntnis zugrunde, dass es nicht ausreicht, wenn das Lebensmittel per se eine geringe Bakterienbelastung aufweist, sondern, dass die auch für die Maschinen, das Verpackungsmaterial und den Werker gelten muss, insbesondere für die Bereiche, die unmittelbar oder zumindest mittelbar mit dem Lebensmittel oder medizintechnischen / pharmakologischen / anderen Hygiene sensitiven Gegenstand in Berührung kommen.

Dies wird dadurch erreicht, dass der Belag von Bakterien und/oder deren Stoffwechselprodukten auf der Oberfläche des Lebensmittelproduktes und/oder der Verarbeitungs- und/oder Verpackungsmaschine und/oder der Schutzkleidung eines Werkers mit Bakterien und/oder deren Stoffwechselprodukten gemessen wird. Sollte einer oder mehrere der gemessenen Werte einen zulässigen Grenzwert überschreiten, kann beispielsweise ein Alarm abgesetzt und/oder das betroffene Produkt ausgeschleust werden. Alternativ oder zusätzlich kann eine außerplanmäßige Reinigung der Verarbeitungs- und/oder Verpackungsmaschine erfolgen und/oder der Werker muss seine Schutzkleidung wechseln.

Bei einer Verpackungsmaschine erfolgt das Messen vorzugsweise:
- am Produkt und/oder an der Verpackung bevor das Produkt in die Verpackung eingelegt ist und/oder nachdem das Produkt eingelegt worden ist,
- nach dem Evakuieren und/oder nach dem Rückbegasen, beispielsweise um eine Kontaminierung durch das Gas zu erkennen,
- innerhalb von Gasleitungen und/oder Schläuchen und/oder Ventilen und/oder Verbindungs- und/oder Versorgungselementen.
- der produktberührenden Seiten von Oberfolie und/oder Unterfolie und/oder - bei mehrlagigen Verpackungen - von Zwischenfolie(n) und/oder des Bedienfelds des Bedienpults, das eine besondere Kontaminationsquelle darstellt, weil es von unterschiedlichen Werkern oftmals angefasst wird und bei der Reinigung vernachlässigt wird
- in der Füllstation / Einlegestation, insbesondere an deren Rand, an der beispielsweise umzupackende Lebensmittel zwischengelagert werden, die für das erneute Verpacken vorgesehen sind; solche Lebensmittel stammen beispielsweise aus fehlgewichtigen Verpackungen, werden daraus entnommen, am Rand der Einlegestation zwischengelagert, deren Gewicht korrigiert, beispielsweise durch Hinzufügen oder Entnehmen von Produktscheiben, bevor oder während sie in der Einlegestation erneut in die noch unverschlossene Verpackung eingelegt werden.

Bei den Verarbeitungsmaschinen stromaufwärts von einer Verpackungsmaschine, beispielsweise einem Kutter, einem Mixer, einem Tumbler, einer Mariniereinheit, einem Slicer, einem Transportsystem, einem Transportbandsystem, einem Handlingroboter, einer Pick & Place-Einheit, einer Waage, einem Produktscanner wird vorzugsweise die produktberührende Oberfläche der Maschine und/oder das Bedienfeld auf Oberflächenbelag gemessen. Beim Slicer erfolgt das Messen vorzugsweise auf dem Slicermesser und/oder dem Aufreihband und/oder den stromabwärts davon nachfolgenden Bändern oder Transporteinrichtungen, mit denen der geschnittene Lebensmittelstapel vorzugsweise in die Verpackungsmaschine transportiert und dort in die Verpackung eingelegt wird. Erfolgt das Einlegen beispielsweise mit dem Greifer an einem Tripod, so erfolgt das Messen vorzugsweise am Greifer, beispielsweise indem der Greifer in zyklischen Abständen in eine Messposition gebracht wird und dort das Messen erfolgt. Nach dem Wechsel des Greifers erfolgt vorzugsweise eine Kontrollmessung.

Bei Maschinen mit Teilen, die regelmäßig ausgetauscht werden müssen, weil sie beispielsweise stumpf sind, wird vorzugsweise die Oberfläche auf Belag vermessen, bevor und/oder nachdem das Austauschteil an der jeweiligen Maschine montiert worden ist.

Bei sich während der Verarbeitung und/oder Verpackung bewegenden Oberflächen kann das Messen im Stillstand der Oberfläche und/oder während deren Bewegung erfolgen.

Beispielsweise kann bei einer Verpackungsmaschine die Oberfläche des Lebensmittelprodukts und/oder des Verpackungsmaterials vor und/oder während des jeweiligen Vorzugs erfolgen; das hat den Vorteil, dass das gesamte Messsystem wirtschaftlicher herstellbar ist, da beispielsweise bei einem Vorzug aus n Reihen und m Spalten nur n Messköpfe benötigt werden, um in allen n Reihen zu messen, wohingegen beim Messen im Stillstand der Oberfläche entsprechend dem Format der Verpackungsmaschine n x m Messköpfe benötigt werden.

Besonders bevorzugt wird nur ein Messkopf benötigt, dessen Messstrahlen beispielsweise über eine nachführbare Spiegeloptik abgelenkt werden und im Stillstand und/oder während der Bewegung der Oberflächen die Messstrahlen so abgelenkt und nachgeführt werden, dass beispielsweise die Messung nacheinander an allen Oberflächen in einem Vorzug erfolgt. Solche nachführbaren Spiegeloptiken sind beispielsweise von Laser-Messsystemen oder -kennzeichnungssystemen bekannt.

Gemäß der Erfindung werden die erzielten Messergebnisse in einem Speichermedium, beispielsweise einem RFID, das sich an einer Verpackung befindet, und/oder in einer Datenbank zusammen mit einer Verpackungsidentifikation abgespeichert. So kann der gemessene Oberflächenbelag in einem Reklamationsfall nachvollzogen werden.

Zusätzlich werden die Messdaten und/oder das Analyseergebnis auf die Verpackung und/oder auf deren Etiketten, vorzugsweise in codierter Form oder auch im Klartext als Beurteilungshilfe für den Verbraucher, insbesondere in Kombination mit dem Messdatum aufgedruckt.

Gemäß einer bevorzugten Ausführungsform der vorliegenden Erfindung wird ein zur Messung eingesetzter Sensor kalibriert. Beispielsweise kann die Referenzmessung an einem Lebensmittel, insbesondere einem ganz frischen Lebensmittel, dessen Keimbefall zusätzlich an der Oberfläche z. B. mit UV-Licht abgetötet werden kann und/oder deren Stoffwechselprodukte deaktiviert/entfernt worden sind, erfolgen. Das Abtöten der Bakterien erfolgt vorzugsweise mit UV-Strahlung, insbesondere bei einer Wellenlänge von 180 - 220 nm, vorzugsweise 195 - 205 nm.

Für den Fall dass die Messung in der geschlossenen Verpackung erfolgt, wird die Kalibrierung vorzugsweise unter Berücksichtigung des Verpackungsmaterials durchgeführt, d.h. bei der Kalibrierung wird der Einfluss des Verpackungsmaterials auf den erhaltenen Messwert berücksichtigt. Das Verpackungsmaterial ist vorzugsweise so ausgeführt, dass es die Messung nicht oder auf reproduzierbare, beispielsweise durch physische oder Softwarefilter korrigierbare Weise behindert, vorzugsweise ist das Verpackungsmaterial im Bereich des Strahlenganges klar bzw. ungetrübt / durchsichtig, kann aber auch eine gewisse Trübung und/oder einen gewissen Farbauftrag, beispielsweise eine Bedruckung, beinhalten, die beispielsweise bei der Kalibrierung und/oder der Intensität des oder der Messstrahlen berücksichtigt wird.

Bei der Verarbeitungs- und/oder Verpackungsmaschine erfolgt die Überprüfung des Reinigungsergebnisses vorzugsweise nach deren Komplettreinigung beispielsweise in der Nachtschicht und/oder an Teilen/Bereichen der jeweiligen Maschine nach einer Zwischenreinigung, z. B. Wischreinigung des Bedienpults und/oder des Einlegebereichs mit Reinigungstüchern. Auch dieses Ergebnis wird vorzugsweise in einem Speicher hinterlegt.

Alternativ oder zusätzlich kann der Hygienezustand der jeweiligen Maschine zu Schichtbeginn oder bei Schichtende überprüft werden.

Vorzugsweise wird der bei Schichtende oder ein anderer gemessener Wert herangezogen, das nachfolgende Reinigungsprogramm festzulegen. Das Reinigungsprogramm wird aufgrund der gemessenen Ergebnisse festgelegt. Nach der Reinigung kann wiederum eine Überprüfung erfolgen, um sicherzustellen, dass die Reinigung hinreichend war.

Ein weiterer Gegenstand der vorliegenden Erfindung ist eine Lebensmittelverarbeitungs- und/oder Verpackungsmaschine, die einen Sensor aufweist, mit dem ein Belag der Oberfläche des Lebensmittelproduktes und/oder der Verarbeitungs- und/oder Verpackungsmaschine und/oder der Schutzkleidung eines Werkers mit Bakterien und/oder deren Stoffwechselprodukten gemessen wird und die erzielten Messergebnisse in einem Speichermedium, das sich an einer Verpackung befindet, und/oder in einer Datenbank zusammen mit einer Verpackungsidentifikation abgespeichert werden.

Die zu diesem Gegenstand der vorliegenden Erfindung gemachten Ausführungen gelten für die anderen Gegenstände der vorliegenden Erfindung gleichermaßen und umgekehrt.

Die erfindungsgemäße Maschine weist einen Sensor auf, mit dem der Belag an Bakterien und/oder deren Stoffwechselprodukten analysiert werden kann. Vorzugsweise ist dieser Sensor ortsfest mit der jeweiligen Maschine verbunden und ist insbesondere an die Energieversorgung der Maschine angeschlossen. Vorzugsweise wird der Sensor beim Reinigen der Maschine abgenommen oder abgedeckt. Vorzugsweise weist die Verpackungsmaschine eine Lichtquelle auf, die insbesondere Licht mit einer Wellenlänge von 390 - 410 nm abstrahlt. Diese Abstrahlung kann punkt- oder flächenförmig erfolgen. Vorzugsweise erfolgt die Abstrahlung so, dass ein Werker davon unbeeinflusst bleibt. Für den Fall dass mehrere Verpackungen gleichzeitig sowie parallel analysiert werden müssen, weist die erfindungsgemäße Maschine vorzugsweise mehrere Sensoren auf, die jeweils eine Verpackung analysieren. Somit kann jede Verpackung einzeln analysiert werden.

Nach dem Messen und/oder simultan während des Messens wird das Messergebnis analysiert, beispielsweis direkt im Messkopf des Sensors und/oder in einer externen Steuerung/Regelung, der Maschine. Das erzielte Ergebnis kann zur Steuerung/Regelung der jeweiligen Maschine oder einer Linie, bestehend aus mehreren Maschinen herangezogen werden. Beispielsweis können Verpackungen und/oder Chargen mit einer zu hohen Bakterienzahl verworfen werden und/oder es kann ein Sterilisierungs- und/oder Reinigungsverfahren, beispielsweise mit Strahlung und/oder einem entsprechenden chemischen Mittel automatisch in Gang gesetzt oder zumindest von einer Steuerung vorgeschlagen werden.

Alternativ oder zusätzlich können Prozessparameter der jeweiligen Maschine durch das Messergebnis anders eingestellt werden. Bei einer Verpackungsmaschine kann beispielsweise in Abhängigkeit vom Messergebnis bzw. Analyseergebnis, die Zusammensetzung der geänderten Atmosphäre geändert werden.

Vorzugsweise erfolgt die Kommunikation zwischen dem Sensor und z. B. der Steuerung/Regelung der Maschine drahtlos, z. B. via WLAN, Bluetooth. Vorzugsweise ist die Maschine an das Intranet des Betriebes angeschlossen, so dass die Messergebnisse auch entfernt von der jeweiligen Maschine verfügbar sind.

Vorzugsweise weist die Maschine ein Bedienpult oder eine Linie einen Leitstand auf. Vorzugsweise wird der Hygienezustand der jeweiligen Maschine an deren Bedienpult und/oder am Leitstand angezeigt. Beispielsweise wird der Zustand der Verpackungsmaschine und/oder von deren Komponenten angezeigt. Weiterhin bevorzugt wird eine Service- und/oder Reinigungsanweisung z. B. aus den Analysedaten abgeleitet und insbesondere am Bedienpult und/oder Leitstand angezeigt.

Vorzugsweise ist die Analysesoftware selbstlernend, indem sie sich beispielsweise durch einen Algorithmus auf die Messaufgabe anhand vorausgegangener Messungen so optimiert und/oder einstellt, dass sie schneller und/oder zielgerichteter auf das Mess- und/oder Analyseergebnis hinführt.

Vorzugsweise erzeugt die Analysesoftware auf Basis einer oder mehrerer, vorzugsweise zeitlich beabstandeter Messungen eine Prognose bzw. Vorhersage des zu einem bestimmten späteren Zeitpunkts zu erwartenden Messergebnisses, beispielsweise im Sinne eines Mindesthaltbarkeitsdatums (MHD).

Vorzugsweise informiert die Analysesoftware mittels geeigneter Meldungen den Bediener über vorhergesagte Messergebnisse und - im Falle von als kritisch einzustufenden Messergebnissen - über mögliche Fehler und/oder Fehlerquellen, die zu den als kritisch einzustufenden Messergebnissen führen, und über typische Abhilfen gegen diese Fehler bzw. Fehlerquellen. Vorzugsweise kann der Bediener selbst aus seinem Erfahrungsschatz die Analysesoftware mit weiteren möglichen Fehler und/oder Fehlerquellen und/oder weiteren typischen Abhilfen füttern, um die Analysesoftware weiter zu verbessern.

Vorzugsweise weist die Maschine einen zusätzlichen Sensor auf, um eine zusätzliche Eigenschaft zu messen, beispielsweise:
- die Temperatur von einem Produkt und/oder einer Verpackung und/oder einer Folie und/oder eines Maschinenbauteils und/oder des Schutzes des Werkers, beispielsweise zum Zweck der Kalibrierung und/oder Erreichung einer besseren Genauigkeit und/oder Wiederholbarkeit der Messung des Oberflächenbelags und/oder
- einen Farbsensor zur Bestimmung der spektralen Zusammensetzung des sichtbaren und/oder unsichtbaren Lichts, beispielsweise zur Groberfassung des Qualitätszustands von Fleisch anhand von dessen Oberflächenfarbe und/oder zur Erfassung des zurückgestrahlten Fluoreszenzlichts,
- die Gas-Konzentration mindestens eines Gases oder Gasbestandteils in der Verpackung, beispielsweise die Sauerstoffkonzentration, und/oder
- den Gasdruck oder Atmosphärendruck in der Verpackung.

Vorzugsweise erfolgt eine lückenlose insbesondere elektronische Erfassung und Protokollierung der gewonnen Messergebnisse entlang der Logistikkette vom Wareneingang des Vor- und/oder Endprodukts bis zum Verlassen des Werkes im Kartons im LKW mittels intelligentem Warenbegleiter, oder sogar bis zum Ladengeschäft, oder sogar bis zum Verbraucher und dessen Haushalt und/oder Geschäftsbetrieb.

Vorzugsweise erfolgt die Lagersteuerung bzw. das Warenwirtschaftssytem, beispielsweise im Ladengeschäft, hygienegesteuert, indem die Verpackungen nach Auswahl anhand des Messergebnisses zum Verkauf zur Verfügung gestellt werden, beispielsweise in die Auslage des Ladengeschäfts gelegt werden, wobei vorzugsweise zuerst die Verpackungen mit der höchsten Belastung mit schädlichem Oberflächenbelag an Bakterien und/oder deren Stoffwechselprodukten und/oder von biochemischen Reaktionsprodukten in die Auslage gelegt werden, und das Legen in die Auslage nicht nach dem Prinzip first-in first-out erfolgt. So wird sichergestellt, dass die Verpackungen mit der höchsten Belastung und/oder die zum frühesten Verderben des Lebensmittel führen würden, zuerst verkauft und zumeist beim Verbraucher zuerst verbraucht werden und somit der Abfall an verdorbenen Lebensmitteln reduziert bzw. der Ressourcenverbrauch optimiert werden.

Vorzugsweise kann der Verbraucher an der Registrierkasse und/oder Kontrollwaage im Ladengeschäft eine Kontrollmessung vornehmen oder vornehmen lassen, wobei das Ergebnis der Messung beispielsweise ergibt, ob das auf der Verpackung angegebene Mindesthaltbarkeitsdatum gemäß dem aktuellen Hygienezustand, also der Belastung mit Oberflächenbelag, tatsächlich so erwartet werden darf oder nicht.

Im Zusammenhang mit der Erfindung ist ein Messgerät zum Messen eines Oberflächenbelags an Bakterien und/oder deren Stoffwechselprodukten auf einem Lebensmittel und/oder auf einer Lebensmittelverarbeitungs- und/oder einer Verpackungsmaschine, wobei es sich um ein Mittel mit einem Multipixelsensor, beispielsweise eine CCD-Kamera handelt, die vorzugsweise mit Farbfiltern zur Begrenzung und/oder Verstärkung zumindest von Teilen des Spektralbereichs des Lichts ausgestattet ist.

Das angewandte Messprinzip beruht auf:
1) Die zu analysierende Oberfläche wird mit UV-Strahlung, vorzugsweise im Wellenlängenbereich zwischen 395 und 415 nm, insbesondere 405 nm angeregt.
2) Die Wellenlänge des zurückgestreuten Lichts ändert sich je nach Zustand des Fleisches, weil die UV-Strahlung bestimmte Stoffwechselprodukte der Bakterien auf der zu analysierenden Oberfläche, beispielsweise des Fleisches fluoreszieren und/oder phosphoreszieren lässt.

Erfindungsgemäß wird die zurückgestrahlte Strahlung von einem Mittel, insbesondere einer Kamera, mit Multipixelsensor empfangen und mit einer entsprechenden Software, beispielsweise einer Bilderkennungssoftware und/oder Spektralanalysesoftware analysiert. Vor dem Multiplexsensor kann ein Lichtfilter vorgesehen sein, der nur einen bestimmten Wellenbereich, insbesondere den oben angegebenen Wellenbereich durchlässt.

Die Anregungslichtquelle ist vorzugsweise als Punkt- und/oder Flächenlichtquelle, z. B. UV-Licht, beispielsweise als Laser- und/oder LED-Licht und/oder Gasentladungslicht, vorzugsweise mit Pixelaufbau, besonders bevorzugt mit einer Steuerung/Regelung der Intensität jedes einzelnen oder einer Gruppe von Pixel, insbesondere zwecks Flächenanreger oder pixelweise Anregung, ausgeführt.

Vorzugsweise ist das Mittel zur Messung modular aufgebaut, beispielsweise zum Austausch von Optik/Optikbaugruppen oder des Sensorchips zur Anpassung des Sensors an das Messobjekt und/oder dessen Eigenschaften/Beschaffenheitsparameter. Vorzugsweise wird das Mittel von der jeweiligen Maschine oder von der Liniensteuerung gesteuert und/oder geregelt, beispielsweise aktiviert.

Vorzugsweise lässt sich das Mittel zur Messung wie folgt einsetzen:
- optische Fluoreszenzanalyse bzw. Farberkennung,
- es können ein einzelner Messpunkt oder mehrere Messpunkte und/oder einzelne Messfläche/Messfleck oder mehrere Messflächen/Messflecke und/oder vorzugsweise die ganze Oberfläche des zu verarbeitenden und/oder zu verpackenden Produkts und/oder von dessen Bestandteilen, beispielsweise von Zusatzstoffen und Additiven (z. B. Marinaden, Panaden) und/oder von Folgeprodukten, beispielsweise von Fertiggerichten und/oder der Verpackung und/oder von Hilfsstoffen, beispielsweise von Einlegepads für die Fleischsaftaufnahme und/oder von Maschinenbauteilen/- gruppen und/oder von Menschen (z. B. Hände) und/oder von Hilfsmitteln (z. B. Handschuhe des Bedienpersonals) und/oder zumindest Bereichen von Verarbeitungs-, Handhabungs- und/oder Verpackungsmaschinen mit dem Messgerät vermessen werden; oder es wird eine Kombinationen von Messflecken zur Verfolgung bestimmter Messstellen eingesetzt.
- Messen der eventuellen gleichverteilten Gesamtkeimbelastung von Lebensmitteln, und/oder lokaler Belastung, die beispielsweise durch das Anfassen mit Handschuhen und/oder kontaminierte Slicermesser und/oder Transportsysteme und/oder durch Gasströme entsteht,
- Messen der Keimbelastung von Flüssigkeiten, beispielsweise von Milch oder Sauce,
- Bestimmen von Qualitätsparametern von Lebensmitteln, beispielsweise vornehmen einer Klassifizierung anhand der Messung, beispielsweise des Anteils an zumeist eiweißfreiem Bindegewebe von einem Fleischstück in hoch, mittel und niedrig.

Vorzugsweise ist die Wellenlänge der Strahlungsquelle veränderbar, insbesondere von ca. 200 nm auf ca. 400 nm. Dadurch kann der Sensor zur Detektion von Bakterien bzw. deren Stoffwechselprodukten und zum Abtöten von Bakterien eingesetzt werden.

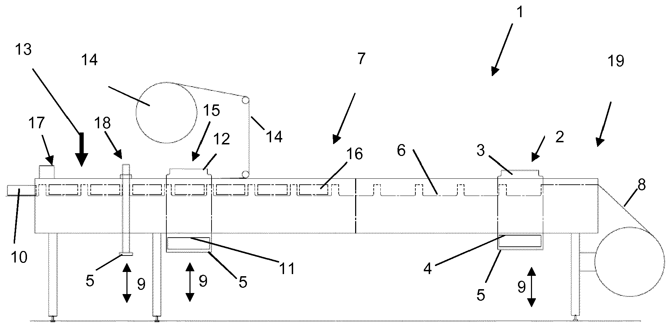

Im Folgenden wird die Erfindung anhand der Figur 1 erläutert. Diese Erläuterungen sind lediglich beispielhaft und schränken den allgemeinen Erfindungsgedanken nicht ein. Die Erläuterungen gelten für alle Gegenstände der vorliegenden Erfindung gleichermaßen.
- **Figur 1**: zeigt die erfindungsgemäße Verpackungsmaschine

**Figur 1** zeigt die erfindungsgemäße Verpackungsmaschine 1, die eine Tiefziehstation 2, eine Füllstation 7 sowie eine Siegelstation 15 aufweist. Eine Kunststofffolienbahn 8, die sogenannte Unterfolienbahn, wird von einer Vorratsrolle abgezogen und, vorzugsweise taktweise, entlang der erfindungsgemäßen Verpackungsmaschine hiervon rechts nach links transportiert. Bei einem Takt wird die Folienbahn um eine Formatlänge weitertransportiert. Dafür weist die Verpackungsmaschine zwei Transportmittel (nicht dargestellt), in dem vorliegenden Fall jeweils zwei Endlosketten auf, die rechts und links von der Folienbahn angeordnet sind. Jede Endloskette weist Haltemittel auf, die jeweils mit einer Kante der Folienbahn zusammenwirken. Sowohl am Anfang als auch am Ende der Verpackungsmaschine ist für jede Kette jeweils mindestens ein Zahnrad vorgesehen, um das die jeweilige Kette umgelenkt wird. Mindestens eines dieser Zahnräder ist angetrieben. Die Zahnräder im Einlaufbereich 19 und/oder im Auslaufbereich können miteinander, vorzugsweise durch eine starre Welle, verbunden sein. Jedes Transportmittel weist eine Vielzahl von Klemmmitteln auf, die die Unterfolienbahn 8 im Einlaufbereich klemmend ergreifen und die Bewegung des Transportmittels auf die Unterfolienbahn 8 übertragen. Im Auslaufbereich der Verpackungsmaschine wird die klemmende Verbindung zwischen dem Transportmittel und der Unterfolienbahn wieder gelöst. In der Tiefziehstation 2, die über ein Oberwerkzeug 3 und ein Unterwerkzeug 4 verfügt, das die Form der herzustellenden Verpackungsmulde aufweist, werden die Verpackungsmulden 6 in die Unterfolienbahn 8 eingeformt. Das Unterwerkzeug 4 ist auf einem Hubtisch 5 angeordnet, der, wie durch den Doppelpfeil symbolisiert wird, vertikal verstellbar ist. Vor jedem Folienvorschub wird das Unterwerkzeug 4 abgesenkt und danach wieder angehoben. Im weiteren Verlauf der Verpackungsmaschine werden die Verpackungsmulden dann in der Füllstation 7 mit dem Verpackungsgut 16 gefüllt. In der sich daran anschließenden Siegelstation 19, die ebenfalls aus einem Oberwerkzeug 12 und einem vertikal verstellbaren Unterwerkzeug 11 besteht, wird eine Oberfolienbahn auf die Verpackungsmulde gesiegelt. Auch in der Siegelstation werden das Oberwerkzeug und/oder das Unterwerkzeug vor und nach jedem Folientransport abgesenkt bzw. angehoben. Auch die Oberfolienbahn 14 kann tiefgezogen und/oder in Transportmitteln geführt sein bzw. von Transportketten transportiert werden, wobei sich diese Transportmittel dann nur von der Siegelstation und ggf. stromabwärts erstrecken. Ansonsten gelten die Ausführungen, die zu dem Transportmitteln der Unterfolienbahn gemacht wurden. In der Siegelstation findet vorzugsweise ein Gasaustausch statt, um beispielsweise den Sauerstoffgehalt der Atmosphäre in der Verpackung zu reduzieren. Im weiteren Verlauf der Verpackungsmaschine werden auch die fertiggestellten Verpackungen vereinzelt, was mit dem Schneidwerkzeugen 17, 18 erfolgt. Das Schneidwerkzeug 18 ist in dem vorliegenden Fall ebenfalls mit einer Hubeinrichtung 9 anhebbar bzw. absenkbar. Der Fachmann erkennt, dass bei einem Takt vorzugsweise mehrere Verpackungsmulden tiefgezogen, befüllt und verschlossen werden.

Die erfindungsgemäße Verpackungsmaschine weist zumindest ein Messgerät, beispielsweise einen Sensor 13 auf, mit dem ein Belag an Bakterien und/oder deren Stoffwechselprodukte auf dem Verpackungsgut und/oder dem Verpackungsmaterial analysiert wird. Dafür wird die zu analysierende Oberfläche mit einer UV-Strahlung angeregt und die fluoreszierende Rückstrahlung der Oberfläche analysiert. Vorzugsweise werden alle hergestellten Verpackungen analysiert. Dafür kann es nötig sein, dass mehrere Sensoren und/oder Strahlungsquellen parallel bezogen auf die Transportrichtung der Folienbahn vorgesehen sind. Die Analyse kann bei dem Folienvorzug und/oder danach erfolgen. Die gewonnen Messergebnisse werden vorzugsweise verpackungsindividuell abgespeichert, zur Steuerung der Verpackungsmaschine und/oder zum Ausschleusen einzelner Verpackungen. Die Verpackungsmaschine kann Teil einer Linie sein, die weitere Maschinen stromaufwärts und/oder stromabwärts aufweist. Eine oder mehrere dieser Maschinen können einen Sensor 13 aufweisen.

### Bezugszeichenliste:

- 1: Verpackungsmaschine
- 2: Tiefziehstation
- 3: Oberwerkzeug der Tiefziehstation
- 4: Unterwerkzeug der Tiefziehstation
- 5: Hubtisch, Träger eines Werkzeugs der Siegel- , Tiefziehstation und/oder der Schneideinrichtung
- 6: Verpackungsmulde
- 7: Füllstation, Einlegestation
- 8: Unterfolienbahn
- 9: Hubeinrichtung
- 10: fertiggestellte Verpackung
- 11: Unterwerkzeug der Siegelstation
- 12: Oberwerkzeug der Siegelstation
- 13: Sensor, Sauerstoffsensor, Mittel, Mittel zur Messung, Lichtquelle, Strahlungsquelle, Farbsensor
- 14: Oberfolie
- 15: Siegelstation
- 16: Verpackungsgut
- 17: Längsschneider
- 18: Querschneider
- 19: Einlaufbereich

## Patentansprüche

1. Verfahren zum Verarbeiten und/oder Verpacken eines Lebensmittelproduktes und/oder medizintechnischen Produkts und/oder pharmakologischen Produkts und/oder anderen Hygiene sensitiven Produkts mit einer Verarbeitungs-, Handhabungs- und/oder Verpackungsmaschine, **dadurch gekennzeichnet, dass** ein Belag der Verarbeitungs-, Handhabungs- und/oder Verpackungsmaschine und/oder der Schutzkleidung eines Werkers mit Bakterien und deren Stoffwechselprodukten und von biochemischen Reaktionsprodukten mit einer UV-Strahlung angeregt wird, die fluoreszierende Rückstrahlung des Belages gemessen wird und die erzielten Messergebnisse in einem Speichermedium, das sich an einer Verpackung befindet, und/oder in einer Datenbank zusammen mit einer Verpackungsidentifikation abgespeichert werden.

2. Verarbeitungs- und/oder Handhabungs- und/oder Verpackungsmaschine, **dadurch gekennzeichnet, dass** sie einen Sensor aufweist, mit dem ein Belag der Oberfläche der Verarbeitungs- und/oder Handhabungs- und/oder Verpackungsmaschine und/oder der Schutzkleidung eines Werkers mit Bakterien und deren Stoffwechselprodukten unter UV-Strahlung-Anregung die fluoreszierende Rückstrahlung des Belages gemessen wird und die erzielten Messergebnisse in einem Speichermedium, das sich an einer Verpackung befindet, und/oder in einer Datenbank zusammen mit einer Verpackungsidentifikation abgespeichert werden.

## Claims

1. Method for processing and/or packaging a food product and/or medical product and/or pharmacological product and/or other hygiene-sensitive product with a processing, handling and/or packaging machine, **characterized in that** a deposit of bacteria and the metabolic products thereof and of biochemical reaction products on the processing, handling and/or packaging machine and/or the protective clothing of a worker is excited with a UV radiation, the fluorescent reflection from the deposit is measured and the measurement results achieved are stored together with a packaging identification in a storage medium that is located on a packaging and/or in a database.

2. Processing and/or handling and/or packaging machine, **characterized in that** it has a sensor, with which a deposit of bacteria and the metabolic products thereof on the surface of the processing and/or handling and/or packaging machine and/or the protective clothing of a worker under excitation with UV radiation the fluorescent reflection of the deposit is measured and the measurement results achieved are stored together with a packaging identification in a storage medium that is located on a packaging and/or in a database.

## Revendications

1. Procédé pour traiter et/ou emballer un produit alimentaire et/ou un produit de technique médicale et/ou un produit pharmaceutique et/ou d'autres produits sensibles du point de vue de l'hygiène, avec une machine de traitement, manipulation et/ou d'emballage, **caractérisé en ce qu'**un revêtement avec des bactéries et leurs produits métaboliques, et par des produits de réactions biochimiques, de la machine de traitement, de manipulation et/ou d'emballage et/ou du vêtement de protection d'un opérateur, est excité avec un rayonnement UV, la réflexion fluorescente du revêtement est mesurée et les résultats de mesure obtenus sont mémorisés dans un support de mémoire qui se trouve sur un emballage, et/ou dans une banque de données conjointement avec une identification de l'emballage.

2. Machine de traitement et/ou de manipulation et/ou d'emballage, **caractérisée en ce qu'**elle présente un capteur avec lequel un revêtement, avec des bactéries et leurs produits métaboliques, de la surface de la machine de traitement et/ou de manipulation et/ou d'emballage et/ou du vêtement de protection d'un opérateur, par excitation aux UV, la réflexion fluorescente du revêtement est mesurée et les résultats de mesure obtenus sont mémorisés dans un support de mémoire qui se trouve sur un emballage et/ou dans une banque de données conjointement avec une identification de l'emballage.
